# FASCICULE DE BREVET EUROPEEN

(11) **EP 3 551 605 B1**
(45) Date de publication et mention de la délivrance du brevet: **21.04.2021**
(21) Numéro de dépôt: 17817000.7
(22) Date de dépôt: 07.12.2017
(51) Int. Cl.: C07C 67/03, B01F 5/06, C07C 213/10, C07C 213/06, C07C 219/08, C07C 69/54, B01F 5/10

(54) **PROCEDE DE FABRICATION D'ESTERS (METH)ACRYLIQUES**
VERFAHREN ZUR HERSTELLUNG VON (METH)ACRYLSÄUREESTERN
PROCESS FOR PRODUCING (METH)ACRYLIC ESTERS

(30) Priorité: 08.12.2016 FR 1662132
(43) Date de publication de la demande: 16.10.2019
(73) Titulaire: Arkema France, 92700 Colombes (FR)
(72) Inventeur: DUBUT, Fanny, 57070 Metz (FR); ESCH, Marc, 57450 Theding (FR); GRAIRE, Coralie, 69493 PIERRE-BENITE Cedex (FR); RIFLADE, Benoit, 33430 Bazas (FR)
(74) Mandataire: Arkema Patent
(86) Numéro de dépôt international: PCT/FR2017/053449
(87) Numéro de publication internationale: WO 2018/104677

(56) Documents cités:
- EP-A1- 1 219 587
- WO-A1-2006/040470
- WO-A1-2013/045786
- WO-A1-2013/110877
- WO-A1-2014/096648
- WO-A1-2014/131970
- WO-A1-2016/016528
- WO-A1-2016/124837
- FR-A1- 2 811 986
- ALVES M H ET AL: "Polymer-supported titanate as catalyst for the transesterification of acrylic monomers", COMPTES RENDUS - CHIMIE, ELSEVIER, PARIS, FR, vol. 13, no. 10, 1 octobre 2010 (2010-10-01), pages 1301-1307, XP027298826, ISSN: 1631-0748 [extrait le 2010-09-18]

## Description

### Domaine technique

La présente invention concerne la fabrication d'esters (méth)acryliques selon un procédé en continu par transestérification, et en particulier la fabrication d'acrylate de N,N-diméthylaminoéthyle (désigné ci-après par ADAME).

L'invention fournit un procédé pour produire un ester (méth)acrylique avec une productivité améliorée, par transestérification d'un (méth)acrylate d'alkyle léger, avec un alcool lourd. L'amélioration du procédé de l'invention est basée sur l'optimisation de la section réactionnelle, en particulier au niveau de l'introduction des réactifs et du catalyseur dans le réacteur de transestérification.

### Art antérieur et problème technique

Les procédés industriels de fabrication d'esters (méth)acryliques par transestérification mettent enjeu un (méth)acrylate d'alkyle à chaine « courte » en C₁-C₄, dit (méth)acrylate d'alkyle léger ou (méth)acrylate léger, qui réagit sur un alcool à chaine carbonée plus « longue », dit alcool lourd, en présence généralement d'un catalyseur et d'inhibiteurs de polymérisation, selon la formule (1) générale suivante :

H₂C=C(R)COOR₁ + R₂OH ⇆ H₂C=C(R)COOR₂ + R₁OH (1)

avec R = H ou CH₃ ; R₁ chaine alkyle en C₁-C₄ ; R₂OH alcool lourd

Afin de déplacer l'équilibre vers la formation de (méth)acrylate d'alkyle à chaine « longue », l'alcool léger R₁OH libéré au cours de la réaction est éliminé en continu sous forme d'un azéotrope avec le (méth)acrylate léger en excès. Du fait de la présence d'alcool léger, cet azéotrope est avantageusement recyclé sur l'unité de fabrication du (méth)acrylate léger dont la synthèse est basée sur l'estérification directe de l'acide (méth)acrylique avec l'alcool léger.

La réaction de transestérification s'accompagne de réactions secondaires produisant des impuretés dans le milieu réactionnel, généralement sous la forme de composés « lourds ». Le milieu réactionnel est ensuite soumis à un ensemble de traitements visant à récupérer l'ester (méth)acrylique pur, et à séparer des fractions valorisables.

Dans un souci de productivité, différents travaux ont déjà porté sur le recyclage de fractions générées au cours de la purification, ou sur le traitement de fractions « lourdes » susceptibles de libérer des produits nobles tels que les réactifs mis enjeu dans la réaction. De tels procédés de valorisation de produits nobles sont par exemple décrits dans les documents WO 2013/045786 ou WO 2016/124837.

En effet, le bilan économique global de ces procédés industriels est fortement lié à la mise au mille des matières premières, c'est-à-dire à la quantité de réactifs et de catalyseur nécessaire à l'obtention de 1000 kg de produit final.

Le recyclage de certaines fractions générées lors du traitement de purification permet d'améliorer le bilan matières premières, mais nécessite néanmoins un apport d'énergie important.

Il subsiste donc encore un besoin d'améliorer la productivité des procédés de fabrication des esters (méth)acryliques par transestérification.

Dans le document WO 2006/040470, il est proposé d'effectuer la réaction de transestérification dans un réacteur à déplacement continu d'équilibre, plus précisément un réacteur piston composé d'un échangeur tube horizontal à calandre chicanée dans lequel chaque compartiment peut être assimilé à un réacteur unique parfaitement agité. Selon ce procédé, il est possible d'obtenir une meilleure conversion du réactif alcool, et une meilleure sélectivité de l'ester (méth)acrylique.

Dans le document EP 1 219 587, le mélange des réactifs passe de manière ascendante à travers un lit de résine cationique comme catalyseur dans une boucle de recirculation qui est associée à une cuve agitée assurant ainsi le mélange des réactifs. Ce procédé concerne en particulier la synthèse d'esters (méth)acryliques par estérification directe.

Les inventeurs ont maintenant découvert qu'en répartissant le catalyseur de transestérification de manière plus efficace et plus homogène au sein du milieu réactionnel, il est possible d'améliorer la sélectivité de la réaction et en conséquence la productivité globale d'un procédé de synthèse d'esters (méth)acryliques.

### Résumé de l'invention

La présente invention a donc pour objet un procédé de fabrication en continu d'un ester (méth)acrylique par réaction de transestérification entre un (méth)acrylate d'alkyle léger, choisi parmi le (méth)acrylate de méthyle et le (méth)acrylate d'éthyle, avec un alcool lourd en présence d'un catalyseur, l'alcool lourd étant un alcool linéaire ou ramifié, primaire ou secondaire, comprenant entre 4 et 12 atomes de carbone, pouvant comporter au moins un atome d'azote, caractérisé en ce que les flux alimentant le réacteur sont introduits par l'intermédiaire d'un mélangeur statique qui est un élément de tuyauterie comprenant des hélices ou des chicanes ou tout autre obstacle visant à augmenter la turbulence, placé sur une boucle de recirculation des réactifs, du catalyseur et des flux recyclés en amont du réacteur .

Par « flux alimentant le réacteur », on entend les flux de produits purs (réactifs et catalyseur) ainsi que les flux recyclés dans le procédé.

« Par l'intermédiaire d'un mélangeur statique » signifie que les flux ne sont pas introduits séparément et directement dans le réacteur par exemple à l'aide d'une canne plongeante., Selon l'invention, les flux sont regroupés en un flux unique qui traverse un mélangeur statique constitué d'un élément de tuyauterie comprenant des hélices ou des chicanes ou tout autre obstacle visant à augmenter la turbulence, dans le but d'homogénéiser ce flux avant d'alimenter le réacteur.

Selon l'invention, l'utilisation d'un mélangeur statique pour introduire les matières premières et le catalyseur dans le réacteur permet un meilleur contact des réactifs avec le catalyseur et une meilleure homogénéisation du milieu réactionnel. On évite ainsi la formation de zones à fortes concentrations de catalyseur qui sont propices à la génération de sous-produits lourds. Ces sous-produits sont par exemple des adduits de Michael résultant de réactions d'addition sur les doubles liaisons (méth)acryliques, ou des oligomères ou polymères. On évite aussi la formation de zones à faibles concentrations en catalyseur qui risquent de limiter la conversion des réactifs. Une cinétique uniforme de la conversion des réactifs est alors assurée.

La formation de l'ester (méth)acrylique est favorisée et en conséquence, la sélectivité de la réaction de transestérification est améliorée. Cela entraine une baisse des mises au mille des réactifs et du catalyseur dans le procédé.

Selon l'invention, le mélangeur statique est un élément de tuyauterie comprenant des hélices ou des chicanes ou tout autre obstacle visant à augmenter la turbulence et donc à favoriser le mélange des flux et le mélangeur statique est placé sur une boucle de recirculation des réactifs et du catalyseur et des flux recyclés en amont du réacteur.

Selon un mode de réalisation, le mélangeur statique est placé en amont du rebouilleur équipant le réacteur.

Selon un mode de réalisation, le mélangeur statique est placé en aval du rebouilleur équipant le réacteur.

Selon un mode de réalisation de l'invention, l'alcool lourd est un aminoalcool de formule (II) :

HO-A-N(R'₂)(R'₃) (II)

dans laquelle
- A est un radical alkylène, linéaire ou ramifié, en C₁-C₅
- R'₂ et R'₃, identiques ou différents l'un de l'autre, représentent chacun un radical alkyle en C₁-C₄.

L'alcool lourd peut être par exemple le N,N-diméthyl aminoéthanol (DMAE), le N,N-diéthyl aminoéthanol, le N,N-diméthyl aminopropanol.

Selon un mode de réalisation préféré de l'invention, l'aminoalcool est le N,N-diméthyl aminoéthanol (DMAE), et l'ester (méth)acrylique est l'acrylate de N,N-diméthyl aminoéthyle (ADAME).

Selon un mode de réalisation de l'invention, l'alcool lourd est un alcool de formule R₂OH dans laquelle R₂ représente une chaine alkyle linéaire ou ramifiée en C₄-C₁₂, de préférence en C₅-C₁₂, L'alcool lourd peut être primaire ou secondaire.

L'alcool lourd est par exemple le 2-éthyl hexanol, le 2-octanol ou le 2-propyl heptanol.

Selon l'invention, le (méth)acrylate d'alkyle léger est choisi parmi le (méth)acrylate de méthyle et le (méth)acrylate d'éthyle.

Selon un mode de réalisation préféré de l'invention, le (méth)acrylate d'alkyle léger est l'acrylate de méthyle ou l'acrylate d'éthyle.

Un second objet de l'invention est un procédé de fabrication en continu d'un ester (méth)acrylique par réaction de transestérification entre un (méth)acrylate d'alkyle léger, choisi parmi le (méth)acrylate de méthyle et le (méth)acrylate d'éthyle, avec un alcool lourd en présence d'un catalyseur, l'alcool lourd étant un alcool linéaire ou ramifié, primaire ou secondaire, comprenant entre 4 et 12 atomes de carbone, pouvant comporter au moins un atome d'azote, ledit procédé comprenant au moins les étapes suivantes :
a) l'alimentation d'un réacteur avec un (méth)acrylate d'alkyle léger, un alcool lourd, un catalyseur de transestérification, et au moins un inhibiteur de polymérisation dans un réacteur, et la soumission du mélange réactionnel à des conditions de transestérification pour former :
   i) un mélange de produits comprenant l'ester (méth)acrylique formé, le (méth)acrylate d'alkyle léger et l'alcool lourd non réagis, le catalyseur, les inhibiteurs de polymérisation et des sous-produits lourds ; et
   ii) un mélange azéotropique (méth)acrylate d'alkyle léger / alcool léger libéré ;
b) la distillation sur une première colonne de distillation du mélange i) de produits séparant en tête un flux composé essentiellement de l'ester (méth)acrylique recherché et des produits légers, et en pied une fraction lourde comprenant essentiellement le catalyseur, les inhibiteurs de polymérisation et les sous-produits lourds ;
c) la purification dudit flux de tête à l'aide d'au moins une seconde colonne de distillation, permettant d'obtenir l'ester (méth)acrylique purifié, et un flux de produits légers qui est recyclé à la réaction ;
d) le passage d'au moins une partie de ladite fraction lourde de pied sur un évaporateur à film séparant des traces de composés légers qui sont recyclés à l'alimentation de la première colonne de distillation, le résidu lourd étant éliminé ;
e) éventuellement le recyclage dans le réacteur d'au moins une partie de ladite fraction lourde de pied de la première colonne de distillation ou du résidu lourd formé à l'étape d) ;
f) éventuellement le recyclage du mélange azéotropique ii) formé à l'étape a), à l'unité de production du méth)acrylate d'alkyle léger ;
g) éventuellement le craquage thermique d'au moins une partie de ladite fraction lourde de pied de la première colonne de distillation, ou du résidu lourd formé à l'étape d) ;
   ledit procédé étant caractérisé en ce que les flux alimentant le réacteur passent dans un mélangeur statique qui est un élément de tuyauterie comprenant des hélices ou des chicanes ou tout autre obstacle visant à augmenter la turbulence, placé sur une boucle de recirculation du réacteur.

Selon un mode de réalisation de l'invention, l'étape de purification c) est réalisée à l'aide de deux colonnes de distillation en série.

L'invention est avantageusement mise en œuvre pour la production de l'acrylate de N,N-diméthyl aminoéthyle (ADAME) par réaction de transestérification entre l'acrylate d'éthyle (AE) et le N,N-diméthyl aminoéthanol (DMAE), le mélangeur statique pouvant être placé sur une boucle de recirculation du réacteur en amont ou en aval du rebouilleur équipant le réacteur.

Ainsi, l'invention porte également sur l'utilisation d'un mélangeur statique pour introduire de façon homogène les matières premières et le catalyseur, ainsi qu'éventuellement les flux recyclés, dans la section réactionnelle d'un procédé de production d'acrylate de N,N-diméthyl aminoéthyle (ADAME) par réaction de transestérification entre l'acrylate d'éthyle (AE) et le N,N-diméthyl aminoéthanol (DMAE).

L'invention va maintenant être décrite plus en détails dans la description qui suit, et en référence aux Figures suivantes :
- Figure 1 : illustration d'un mélangeur utilisable selon l'invention.
- Figure 2 : schéma d'une section réactionnelle incluant le mode d'introduction des flux dans un réacteur de transestérification selon l'invention.
- Figure 3 : représentation schématique d'une installation adaptée pour mettre en œuvre le procédé de l'invention.

### Exposé détaillé de l'invention

Dans les procédés de l'art antérieur, le catalyseur est introduit directement dans le réacteur par exemple à l'aide d'une canne plongeante, séparée de l'alimentation des flux de réactifs (comme représenté par exemple dans les documents WO 2013/110877 ; WO 2014/131970 ; FR 2 811986).

Selon la figure illustrant la purification à l'aide d'une seule colonne de distillation, d'un mélange réactionnel issu d'une réaction de transestérification, décrite dans le document WO2014/096648, les réactifs ainsi que le catalyseur sont introduits dans le réacteur sous forme d'un flux unique formé dans une boucle de recirculation. Cependant, l'homogénéisation du milieu réactionnel et la cinétique uniforme de la conversion des réactifs ne sont pas des problèmes dans ce procédé qui concerne essentiellement l'obtention d'un produit final de haute pureté. Il n'est donc nullement suggéré de placer un mélangeur statique dans la boucle de recirculation.

Selon l'invention, on optimise l'introduction des réactifs et du catalyseur en utilisant un mélangeur statique dans la boucle de recirculation du réacteur. Ainsi, on s'assure que le mélange des réactifs et du catalyseur est bien effectué lorsqu'il rentre dans le réacteur après la boucle de recirculation. On évite la présence de surconcentration du catalyseur dans la boucle et dans le réacteur, et une meilleure répartition du catalyseur est réalisée dans le milieu réactionnel.

Comme mélangeurs statiques utilisables, on peut citer les mélangeurs commercialisés par exemple par la société CTMI à St Avold ou par la société Sulzer.

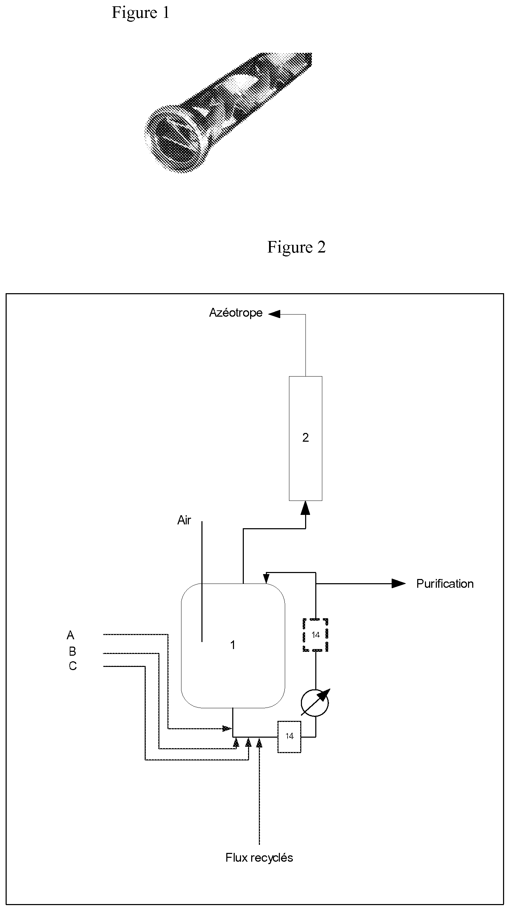

La Figure 1 illustre un exemple de mélangeur adapté pour le procédé selon l'invention.

Sur la Figure 2 est schématisée une section réactionnelle qui comporte un réacteur de transestérification 1 et un mélangeur statique 14. Un flux A de (méth)acrylate d'alkyle et un flux B d'alcool lourd, ainsi qu'un flux C de catalyseur sont regroupés en un flux unique qui traverse un mélangeur statique 14 qui est relié en sortie au réacteur 1. Le mélange intime du catalyseur C avec les réactifs A et B est ainsi réalisé au sein du mélangeur statique, avant d'être introduit dans le réacteur.

Le mélangeur statique 14 peut être placé en amont ou en aval du rebouilleur dans la boucle de recirculation du réacteur.

La formation de sous-produits lourds étant minimisée en raison de l'absence de zones à fortes concentrations de catalyseur dans le réacteur, les conditions réactionnelles peuvent être adaptées afin d'optimiser le rendement de la réaction, notamment en augmentant le temps de séjour dans le réacteur pour conduire à une augmentation des taux de conversion des matières premières, et en conséquence une baisse de la mise au mille des matières premières.

On conduit généralement la réaction de transestérification dans le réacteur 1 à une pression comprise entre 500 mm Hg (67 kPa) et la pression atmosphérique, de préférence sous une pression d'environ 700 mm Hg, (93 kPa) et à une température allant de 70°C à 130°C, de préférence de 100°C à 120°C.

Le rapport molaire (méth)acrylate d'alkyle léger / alcool lourd peut aller de 1 à 3, de préférence il est compris entre 1,3 et 1,8.

La réaction de transestérification est effectuée en présence d'un catalyseur selon les méthodes bien connues de l'homme du métier. Par exemple, le catalyseur de transestérification peut être un titanate de tétraalkyle en solution dans un alcool tel que l'alcool lourd utilisé dans la réaction, de préférence du titanate de tétraéthyle en solution dans l'alcool lourd ; le catalyseur peut être aussi un titanate de l'alcool lourd, par exemple du titanate de tétra (diméthylaminoéthyle), ou du titanate de tétra (2-éthylhexyle).

La réaction est effectuée en présence d'un ou plusieurs inhibiteurs de polymérisation qui sont introduits dans le réacteur, à raison de 1000 à 5000 ppm par rapport au mélange brut réactionnel. Comme inhibiteurs de polymérisation utilisables, on peut citer par exemple la phénothiazine, l'hydroquinone, l'éther monométhylique d'hydroquinone, le diterbutyl para-crésol (BHT), le TEMPO (2,2,6,6-tétraméthyl-1-piperidinyloxy), le di-tertiobutylcatéchol, ou les dérivés du TEMPO, tel que le 4-hydroxy TEMPO (4-OHTEMPO), seuls ou leurs mélanges en toutes proportions.

Le temps de séjour dans le réacteur est généralement compris entre 5 et 8 heures.

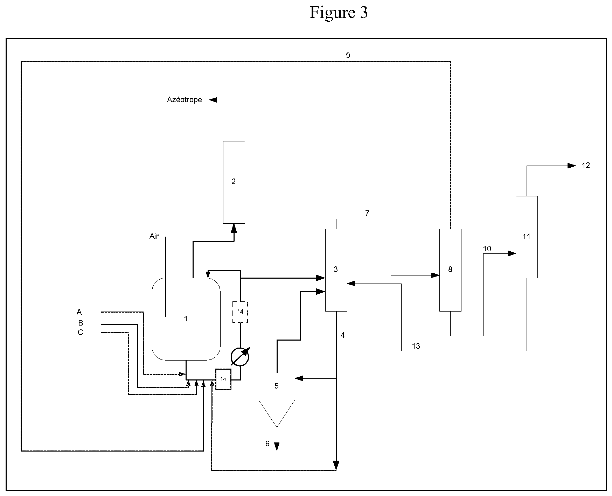

La Figure 3 illustre une installation adaptée pour mettre en œuvre un procédé continu de production d'esters (méth)acryliques par transestérification à partir de (méth)acrylates d'alkyle légers, et d'alcools lourds comme défini dans l'invention.

Cette installation est décrite plus particulièrement et de façon non limitative dans le cas de la fabrication d'ADAME par transestérification à partir d'AE et de DMAE, qui comprend les étapes (a) à (g) telles que définies dans le procédé selon l'invention.

Sur la Figure 3 sont représentés un réacteur de transestérification 1, un rebouilleur et un mélangeur statique 14 qui peut être placé en amont ou en aval du rebouilleur dans une boucle de recirculation du réacteur.

Les réactifs A et B (AE et DMAE) ainsi qu'un catalyseur de transestérification C sont introduits dans la boucle de recirculation du réacteur, de sorte qu'ils sont mélangés de façon homogène avant d'être introduits dans le réacteur 1. Des flux recyclés provenant de la section de purification peuvent être introduits également au niveau de la boucle de recirculation.

Selon une première étape (a), la réaction de transestérification entre l'AE et le DMAE, est effectuée dans le réacteur 1 en présence du catalyseur C, préférentiellement du titanate de tétraéthyle, et d'inhibiteurs de polymérisation. Le réacteur 1 est surmonté d'une colonne de distillation 2 qui sert à éliminer l'alcool léger formé (éthanol) au fur et à mesure de sa formation et à déplacer ainsi l'équilibre réactionnel dans le sens de la formation de l'ADAME.

Le mélange réactionnel comprend l'ADAME formé, l'AE et le DMAE non réagis, le catalyseur C, les inhibiteurs de polymérisation, des adduits de Michael résultant de réactions d'addition sur les doubles liaisons (méth)acryliques, et autres composés lourds tels que oligomères ou polymères.

Selon l'étape (b) du procédé, le mélange réactionnel est soumis à une distillation sur une colonne de distillation (colonne d'équeutage 3). On récupère en tête de la colonne 3, un flux 7 débarrassé de l'essentiel du catalyseur et des inhibiteurs de polymérisation et comportant l'ADAME produit et des composés légers tels que le DMAE, l'AE, avec une fraction minoritaire d'adduits de Michael et de produits lourds.

On récupère en pied de colonne 3 une fraction lourde 4 comprenant le catalyseur, les inhibiteurs de polymérisation, les adduits de Michael et les composés lourds, avec une fraction minoritaire d'ADAME et de DMAE et des traces de composés légers.

Selon l'étape (c) du procédé, le flux 7 est soumis à une purification qui est réalisée à l'aide de la colonne de distillation 8, dont le flux de tête 9 comprenant les réactifs non réagis est recyclé à la réaction, le flux de pied 10 étant dirigé vers une colonne de distillation 11 permettant d'obtenir en tête l'ADAME purifié 12, et en pied un flux 13 riche en inhibiteurs qui est recyclé dans le flux de mélange brut réactionnel alimentant la première colonne 3.

Selon l'étape (d) du procédé, la fraction lourde 4 issue du pied de la colonne 3 qui contient notamment le catalyseur est pour partie concentrée sur un évaporateur à film 5 qui permet de séparer les traces de composés légers qui sont alors recyclés à l'alimentation de la colonne 3, le résidu lourd 6 étant éliminé ou envoyé vers une unité de craquage (non représentée), ou éventuellement il est recyclé à la réaction.

La fraction lourde 4 issue du pied de la colonne 3 peut être en partie recyclée à la réaction (étape e).

La présente invention fournit ainsi une solution simple à mettre en œuvre sur les installations existantes de synthèse d'esters (méth)acryliques et conduisant à une productivité et une sélectivité améliorée. Le procédé selon l'invention minimise également la taille et l'énergie des équipements de séparation/recyclage des flux générés lors de la purification du milieu réactionnel.

Les exemples ci-après illustrent la présente invention sans toutefois en limiter la portée.

### PARTIE EXPERIMENTALE

### Exemple 1

Sur une unité industrielle de production d'ADAME à partir d'acrylate d'éthyle (AE) et de N,N-diméthyl aminoéthanol (DMAE), le catalyseur (solution de titanate d'éthyle dans le DMAE) est introduit à la réaction via une canne plongeante, séparée des flux de réactifs, directement dans le réacteur.

Un mélangeur statique a été placé sur la boucle de recirculation du réacteur comme représenté sur la Figure 2, pour remplacer la canne d'injection. Les réactifs et le catalyseur sont introduits dans le réacteur via ce mélangeur.

Les conditions de réaction, température, temps de séjour et débit, ont été inchangées.

Les mises au mille en alcool DMAE et en catalyseur, ainsi que la quantité de lourds en sortie de l'unité (fraction 6 sur la Figure 3) pour une tonne d'ADAME produite, ont été déterminés sur une période de 8 mois avant et après l'ajout du mélangeur.

Les résultats des essais ont montré que la mise en place d'un mélangeur statique en remplacement des cannes plongeantes pour introduire les réactifs et le catalyseur a conduit à :
Une réduction de 3 kg/t de catalyseur
Une réduction 2 kg/t de DMAE

L'amélioration de la mise au mille AE induite par l'ajout du mélangeur n'a pas été quantifiée.

En outre, la quantité de lourds à la sortie de l'unité a pu être réduite de plus de 10% massique.

La productivité de l'installation a été ainsi globalement augmentée.

### Exemple 2

Afin d'évaluer la qualité du mélange des réactifs au sein d'un réacteur, une méthode de simulation CFD (Computational Fluid Dynamics) a été appliquée. Celle-ci permet de mettre en évidence d'éventuelles zones de faible mélange, apportant une hétérogénéité des temps de séjour qui induit une dégradation de la conversion des réactifs et des sélectivités des réactions chimiques.

Deux approches ont été utilisées pour mettre en évidence les avantages du procédé selon l'invention :

### 1) Visualisation des écoulements et du mélange des réactifs dans un réacteur

Le trajet de particules simulant les composés AE et DMAE à partir de leur introduction dans un réacteur par l'intermédiaire de cannes plongeantes a été visualisé. Une hétérogénéité des parcours suivis par ces réactifs a pu être mise en évidence, la direction suivie par chacun des réactifs n'allant pas dans le sens d'un mélange immédiat.

De plus, selon la disposition choisie dans le réacteur pour la canne d'introduction du DMAE, il a été observé une forte hétérogénéité de sa concentration dans le réacteur en générant des zones enrichies et des zones appauvries en réactif DMAE. Les cannes d'introduction agissent donc comme des obstacles et font elles-mêmes partie des sources de perturbation des flux de réactifs.

La disposition des cannes d'introduction des réactifs dans le réacteur doit faire l'objet d'études d'optimisation pour minimiser ces problèmes.

Selon l'invention, on évite la formation de zones en sur- ou sous- concentration en réactifs, en assurant un parfait mélange des réactifs au moyen d'un mélangeur statique en amont du réacteur, sur la boucle de recirculation. La conversion des réactifs se fait alors selon une cinétique uniforme, et la formation de sous-produits de type Michael est minimisée.

### 2) Détermination du volume efficace dans le réacteur

L'injection de particules des deux réactifs, AE et DMAE, ainsi que du catalyseur, a été simulée dans une cuve, d'une part depuis des canules d'injection, d'autre part depuis la surface libre recevant le flux d'un mélange des réactifs et du catalyseur effectué en amont. On a fait varier le nombre de pas de temps d'injection (un pas est égal 2ms) pour vérifier la vitesse à laquelle les réactifs se répandent dans la cuve. A la fin de l'injection, on a compté le nombre de cellules traversées par l'ensemble des 3 composés, afin de calculer le volume correspondant représentant le volume efficace du réacteur.

La comparaison des 2 systèmes d'injection, exprimée par le pourcentage du volume efficace par rapport au volume du réacteur, est représentée dans le tableau suivant.

| Mode d'introduction des réactifs et catalyseur | Cannes plongeantes | Mélangeur en amont |
|---|---|---|
| 5000 pas de temps | 0% | 27,8 % |
| 10000 pas | 0,3 % | 93,3 % |
| 14000 pas | 50% | 99% |

Avec le procédé selon l'invention, on observe un mélange instantané et homogène des réactifs et les réactifs occupent plus rapidement tout le volume du réacteur.

## Revendications

1. Procédé de fabrication en continu d'un ester (méth)acrylique par réaction de transestérification entre un (méth)acrylate d'alkyle léger choisi parmi le (méth)acrylate de méthyle et le (méth)acrylate d'éthyle, avec un alcool lourd en présence d'un catalyseur, l'alcool lourd étant un alcool linéaire ou ramifié, primaire ou secondaire, comprenant entre 4 et 12 atomes de carbone, pouvant comporter au moins un atome d'azote, **caractérisé en ce que** les flux alimentant le réacteur sont introduits par l'intermédiaire d'un mélangeur statique qui est un élément de tuyauterie comprenant des hélices ou des chicanes ou tout autre obstacle visant à augmenter la turbulence, placé sur une boucle de recirculation des réactifs, du catalyseur et des flux recyclés en amont du réacteur.

2. Procédé selon la revendication 1 **caractérisé en ce que** le mélangeur statique est placé en amont du rebouilleur équipant le réacteur.

3. Procédé selon la revendication 1 **caractérisé en ce que** le mélangeur statique est placé en aval du rebouilleur équipant le réacteur.

4. Procédé selon l'une quelconque des revendications précédentes **caractérisé en ce que** l'alcool lourd est un aminoalcool de formule (II) :
HO-A - N (R'₂)(R'₃) (II)
dans laquelle :
- A est un radical alkylène, linéaire ou ramifié, en C₁-C₅
- R'₂ et R'₃, identiques ou différents l'un de l'autre, représentent chacun un radical alkyle en C₁-C₄.

5. Procédé selon l'une quelconque des revendications précédentes **caractérisé en ce que** l'alcool lourd est le N,N-diméthyl aminoéthanol (DMAE), le N,N-diéthyl aminoéthanol, le N,N-diméthyl aminopropanol, de préférence le N,N-diméthyl aminoéthanol (DMAE).

6. Procédé selon l'une quelconque des revendications 1 à 3 **caractérisé en ce que** l'alcool lourd est un alcool primaire ou secondaire de formule R₂OH dans laquelle R₂ représente une chaine alkyle linéaire ou ramifiée en C₄-C₁₂.

7. Procédé selon l'une quelconque des revendications 1 à 3 **caractérisé en ce que** l'alcool lourd est le 2-éthyl hexanol, le 2-octanol ou le 2-propyl heptanol.

8. Procédé de fabrication en continu d'un ester (méth)acrylique par réaction de transestérification entre un (méth)acrylate d'alkyle léger choisi par le (méth)acrylate de méthyle et le (méth)acrylate d'éthyle, avec un alcool lourd en présence d'un catalyseur, l'alcool lourd étant un alcool linéaire ou ramifié, primaire ou secondaire, comprenant entre 4 et 12 atomes de carbone, pouvant comporter au moins un atome d'azote,
ledit procédé comprenant au moins les étapes suivantes :
a) l'alimentation d'un réacteur avec un (méth)acrylate d'alkyle léger, un alcool lourd, un catalyseur de transestérification, et au moins un inhibiteur de polymérisation, et la soumission du mélange réactionnel à des conditions de transestérification pour former :
i) un mélange de produits comprenant l'ester (méth)acrylique formé, le (méth)acrylate d'alkyle léger et l'alcool lourd non réagis, le catalyseur, les inhibiteurs de polymérisation et des sous-produits lourds ; et
ii) un mélange azéotropique (méth)acrylate d'alkyle léger / alcool léger libéré ;
b) la distillation sur une première colonne de distillation du mélange i) de produits séparant en tête un flux composé essentiellement de l'ester (méth)acrylique recherché et des produits légers, et en pied une fraction lourde comprenant essentiellement le catalyseur, les inhibiteurs de polymérisation et les sous-produits lourds ;
c) la purification dudit flux de tête à l'aide d'au moins une seconde colonne de distillation, permettant d'obtenir l'ester (méth)acrylique purifié, et un flux de produits légers qui est recyclé à la réaction ;
d) le passage d'au moins une partie de ladite fraction lourde de pied sur un évaporateur à film séparant des traces de composés légers qui sont recyclés à l'alimentation de la première colonne de distillation, le résidu lourd étant éliminé ;
e) éventuellement le recyclage dans le réacteur d'au moins une partie de ladite fraction lourde de pied de la première colonne de distillation ou du résidu lourd formé à l'étape d) ;
f) éventuellement le recyclage du mélange azéotropique ii) formé à l'étape a), à l'unité de production du méth)acrylate d'alkyle léger ;
g) éventuellement le craquage thermique d'au moins une partie de ladite fraction lourde de pied de la première colonne de distillation, ou du résidu lourd formé à l'étape d) ;
ledit procédé étant **caractérisé en ce que** les flux alimentant le réacteur passent dans un mélangeur statique qui est un élément de tuyauterie comprenant des hélices ou des chicanes ou tout autre obstacle visant à augmenter la turbulence , placé sur une boucle de recirculation du réacteur.

9. Procédé selon la revendication 8 **caractérisé en ce que** l'ester (méth)acrylique est l'acrylate de N,N-diméthyl aminoéthyle obtenu à partir d'acrylate d'éthyle et de N,N-diméthyl aminoéthanol.

10. Utilisation d'un mélangeur statique pour introduire de façon homogène les matières premières et le catalyseur, ledit mélangeur statique étant placé sur une boucle de recirculation des réactifs, du catalyseur et des flux recyclés en amont du réacteur dans la section réactionnelle d'un procédé de production d'acrylate de N,N-diméthyl aminoéthyle (ADAME) par réaction de transestérification entre l'acrylate d'éthyle (AE) et le N,N-diméthyl aminoéthanol (DMAE).

## Patentansprüche

1. Verfahren zur kontinuierlichen Herstellung eines (Meth)acrylsäureesters durch Umesterungsreaktion zwischen einem leichten Alkyl(meth)acrylat, das aus Methyl(meth)acrylat und Ethyl(meth)acrylat ausgewählt wird, mit einem schweren Alkohol in Gegenwart eines Katalysators, wobei es sich bei dem schweren Alkohol um einen linearen oder verzweigten, primären oder sekundären Alkohol mit zwischen 4 und 12 Kohlenstoffatomen, der mindestens ein Stickstoffatom enthalten kann, handelt, **dadurch gekennzeichnet, dass** die den Reaktor speisenden Ströme über einen statischen Mischer eingetragen werden, bei welchem es sich um ein Rohrleitungselement mit Impellern oder Prallplatten oder einem beliebigen anderen Hindernis zur Erhöhung der Turbulenz, das an einer Reaktantenrezirkulationsschleife angeordnet ist, handelt, wobei der Katalysator und die Ströme stromaufwärts des Reaktors rezykliert werden.

2. Verfahren nach Anspruch 1, **dadurch gekennzeichnet, dass** der statische Mischer stromaufwärts des Aufkochers des Reaktors angeordnet ist.

3. Verfahren nach Anspruch 1, **dadurch gekennzeichnet, dass** der statische Mischer stromabwärts des Aufkochers des Reaktors angeordnet ist.

4. Verfahren nach einem der vorhergehenden Ansprüche, **dadurch gekennzeichnet, dass** es sich bei dem schweren Alkohol um einen Aminoalkohol der Formel (II) handelt:
HO-AQ-N(R'₂) (R'₃) (II)
wobei:
- A für einen linearen oder verzweigten C₁-C₅-Alkylenrest steht und
- R'₂ und R'₃, die gleich oder voneinander verschieden sind, jeweils für einen C₁-C₄-Alkylrest stehen.

5. Verfahren nach einem der vorhergehenden Ansprüche, **dadurch gekennzeichnet, dass** es sich bei dem schweren Alkohol um N,N-Dimethylaminoethanol (DMAE), N,N-Diethylaminoethanol oder N,N-Dimethylaminopropanol, vorzugsweise N,N-Dimethylaminoethanol (DMAE), handelt.

6. Verfahren nach einem der Ansprüche 1 bis 3, **dadurch gekennzeichnet, dass** es sich bei dem schweren Alkohol um einen primären oder sekundären Alkohol der Formel R₂OH handelt, wobei R₂ für eine lineare oder verzweigte C₄-C₁₂-Alkylkette steht.

7. Verfahren nach einem der Ansprüche 1 bis 3, **dadurch gekennzeichnet, dass** es sich bei dem schweren Alkohol um 2-Ethylhexanol, 2-Octanol oder 2-Propylheptanol handelt.

8. Verfahren zur kontinuierlichen Herstellung eines (Meth)acrylsäureesters durch Umesterungsreaktion zwischen einem leichten Alkyl(meth)acrylat, das aus Methyl(meth)acrylat und Ethyl(meth)acrylat ausgewählt wird, mit einem schweren Alkohol in Gegenwart eines Katalysators, wobei es sich bei dem schweren Alkohol um einen linearen oder verzweigten, primären oder sekundären Alkohol mit zwischen 4 und 12 Kohlenstoffatomen, der mindestens ein Stickstoffatom enthalten kann, handelt, wobei das Verfahren mindestens die folgenden Schritte umfasst:
a) Speisen eines Reaktors mit einem leichten Alkyl-(meth)acrylat, einem schweren Alkohol, einem Umesterungskatalysator und mindestens einem Polymerisationsinhibitor und Einwirkenlassen von Umesterungsbedingungen auf die Reaktionsmischung zur Bildung:
i) eines Produktgemischs, das den gebildeten (Meth)acrylsäureester, das nicht umgesetzte leichte Alkyl(meth)acrylat und den nicht umgesetzten schweren Alkohol, den Katalysator, die Polymerisationsinhibitoren und schwere Nebenprodukte umfasst; und
ii) eines azeotropen Gemischs von leichtem Alkyl-(meth)acrylat und freigesetztem leichtem Alkohol;
b) Destillieren von Produkten i) in einer ersten Destillationssäule, wobei am Kopf ein Strom, der im Wesentlichen aus dem gewünschten (Meth)acrylsäureester und leichten Produkten besteht, und am Sumpf eine schwere Fraktion, die im Wesentlichen den Katalysator, die Polymerisationsinhibitoren und die schweren Nebenprodukte umfasst, abgetrennt werden;
c) Reinigen des Kopfstroms mit Hilfe mindestens einer zweiten Destillationssäule, was den gereinigten (Meth)acrylsäureester und einen Strom leichter Produkte, der zur Reaktion rezykliert wird, ergibt;
d) Führen mindestens eines Teils der schweren Sumpffraktion durch einen Filmverdampfer, wobei Spuren von leichten Verbindungen abgetrennt werden, die zur Einspeisung der ersten Destillationssäule rezykliert werden, wobei der schwere Rückstand entfernt wird;
e) gegebenenfalls Rezyklieren mindestens eines Teils der schweren Sumpffraktion aus der ersten Destillationssäule oder des in Schritt d) gebildeten schweren Rückstands in den Reaktor;
f) gegebenenfalls Rezyklieren des in Schritt a) gebildeten azeotropen Gemischs ii) zu der Einheit zur Herstellung des leichten Alkyl(meth)acrylats;
g) gegebenenfalls thermisches Cracken mindestens eines Teils der schweren Sumpffraktion aus der ersten Destillationssäule oder des in Schritt d) gebildeten schweren Rückstands;
wobei das Verfahren **dadurch gekennzeichnet ist, dass** die den Reaktor speisenden Ströme einen statischen Mischer durchlaufen, bei welchem es sich um ein Rohrleitungselement mit Impellern oder Prallplatten oder einem beliebigen anderen Hindernis zur Erhöhung der Turbulenz, das an einer Reaktantenrezirkulationsschleife angeordnet ist, handelt.

9. Verfahren nach Anspruch 8, **dadurch gekennzeichnet, dass** es sich bei dem (Meth)acrylsäureester um ausgehend von Ethylacrylat und N,N-Dimethylaminoethanol erhaltenes N,N-Dimethylaminoethylacrylat handelt.

10. Verwendung eines statischen Mischers zum homogenen Eintragen der Ausgangsstoffe und des Katalysators, wobei der statische Mischer an einer Reaktantenrezirkulationsschleife angeordnet ist, wobei der Katalysator und die Ströme stromaufwärts des Reaktors in den Reaktionsteil eines Verfahrens zur Herstellung von N,N-Dimethylaminoethylacrylat (ADAME) durch Umesterungsreaktion zwischen Ethylacrylat (EA) und N,N-Dimethylaminoethanol (DMAE) rezykliert werden.

## Claims

1. Process for continuous production of a (meth)acrylic ester by transesterification reaction of a light alkyl (meth)acrylate selected from methyl (meth)acrylate and ethyl (meth)acrylate with a heavy alcohol in the presence of a catalyst, the heavy alcohol being a linear or branched, primary or secondary alcohol comprising between 4 and 12 carbon atoms and optionally comprising at least one nitrogen atom, **characterized in that** the flows feeding the reactor are introduced by way of a static mixer which is a piping element comprising impellers or baffles or any other obstacle intended to increase turbulence, which is placed on a recirculation loop for the reagents, the catalyst and the flows recycled upstream of the reactor.

2. Process according to Claim 1, **characterized in that** the static mixer is placed upstream of the reboiler of the reactor.

3. Process according to Claim 1, **characterized in that** the static mixer is placed downstream of the reboiler of the reactor.

4. Process according to any one of the preceding claims, **characterized in that** the heavy alcohol is an amino alcohol of formula (II):
HO-A-N(R'₂)(R'₃) (II)
in which:
- A is a linear or branched C₁-C₅ alkylene radical and
- R'₂ and R'₃, which are identical or different from one another, each represent a C₁-C₄ alkyl radical.

5. Process according to any one of the preceding claims, **characterized in that** the heavy alcohol is N,N-dimethylaminoethanol (DMAE), N,N-diethylaminoethanol or N,N-dimethylaminopropanol, preferably N,N-dimethylaminoethanol (DMAE).

6. Process according to any one of Claims 1 to 3, **characterized in that** the heavy alcohol is a primary or secondary alcohol of formula R₂OH in which R₂ represents a linear or branched C₄-C₁₂ alkyl chain.

7. Process according to any one of Claims 1 to 3, **characterized in that** the heavy alcohol is 2-ethylhexanol, 2-octanol or 2-propylheptanol.

8. Process for continuous production of a (meth)acrylic ester by transesterification reaction of a light alkyl (meth)acrylate selected from methyl (meth)acrylate, ethyl (meth)acrylate with a heavy alcohol in the presence of a catalyst, the heavy alcohol being a linear or branched, primary or secondary alcohol comprising between 4 and 12 carbon atoms and optionally comprising at least one nitrogen atom,
said process comprising at least the following steps:
a) feeding a reactor with a light alkyl (meth)acrylate, a heavy alcohol, a transesterification catalyst, and at least one polymerization inhibitor, and subjecting the reaction mixture to transesterification conditions to form:
i) a product mixture comprising the (meth)acrylic ester formed, the unreacted light alkyl (meth)acrylate and the unreacted heavy alcohol, the catalyst, the polymerization inhibitors, and heavy byproducts; and
ii) an azeotropic mixture of light alkyl (meth)acrylate and light alcohol released;
b) distilling, on a first distillation column, the mixture i) of products, with separation at the top of a stream composed essentially of the desired (meth)acrylic ester and light products, and at the bottom a heavy fraction comprising essentially the catalyst, the polymerization inhibitors and the heavy byproducts;
c) purifying said top stream using at least one second distillation column, to give the purified (meth)acrylic ester, and a light product stream which is recycled to the reaction;
d) passing at least a portion of said heavy bottom fraction through a film evaporator, separating traces of light compounds, which are recycled to the feed of the first distillation column, the heavy residue being removed;
e) optionally recycling to the reactor at least a portion of said heavy bottom fraction from the first distillation column or the heavy residue formed in step d) ;
f) optionally recycling the azeotropic mixture ii) formed in step a) to the unit for producing the light alkyl (meth)acrylate;
g) optionally, thermally cracking at least a portion of said heavy bottom fraction from the first distillation column, or the heavy residue formed in step d);
said process being **characterized in that** the streams feeding the reactor pass through a static mixer which is a piping element comprising impellers or baffles or any other obstacle intended to increase turbulence, which is placed on a recirculation loop of the reactor.

9. Process according to Claim 8, **characterized in that** the (meth)acrylic ester is N,N-dimethylaminoethyl acrylate, obtained from ethyl acrylate and N,N-dimethylaminoethanol.

10. Use of a static mixer for uniformly introducing the starting materials and the catalyst, said static mixer being placed on a recirculation loop for the reactants, the catalyst and the streams recycled upstream of the reactor, in the reaction section of a process for producing N,N-dimethylaminoethyl acrylate (ADAME) by transesterification reaction of ethyl acrylate (EA) with N,N-dimethylaminoethanol (DMAE).
